Europäisches Patentamt

**European Patent Office**

**Office européen des brevets**

(11) Publication number: **0 251 707**
**A1**

## (12) EUROPEAN PATENT APPLICATION

(21) Application number: **87305667.5**

(22) Date of filing: **25.06.87**

(51) Int. Cl.⁴: **G01N 33/557** , G01N 33/543

(30) Priority: **26.06.86 US 878950**

(43) Date of publication of application:
**07.01.88 Bulletin 88/01**

(84) Designated Contracting States:
**AT BE CH DE FR GB IT LI LU NL SE**

(71) Applicant: **ORTHO DIAGNOSTIC SYSTEMS INC.**
**One Johnson & Johnson Plaza**
**New Brunswick New Jersey 08933-7003(US)**

(72) Inventor: **Hansen, Peter W.**
**49 Cedar Street**
**Middleboro Massachusetts 02346(US)**

(74) Representative: **Jones, Alan John et al**
**CARPMAELS & RANSFORD 43 Bloomsbury Square**
**London, WC1A 2RA(GB)**

(54) **Methods for calibration of extended surface in homogeneous rate immunoassays.**

(57) A method useful for homogeneous rate immunoassays employing extended reactive surfaces is provided for identifying and/or correcting for inhomogeneities in such extended surfaces including physical inhomogeneities in the surface forming material and inhomogeneities in the coating and/or activity of the immobilized biologically active material on said surface. The method comprises performing, on the same extended surfaces on which the immunoassay shall be conducted, a calibration assay employing a calibration reagent having a known level of activity and capable of binding to said immobilized biologically active molecule in a manner substantially similar to that of the ligand or ligand binding partner to be determined by the sample immunoassay.

EP 0 251 707 A1

# METHOD FOR CALIBRATION OF EXTENDED SURFACES IN HOMOGENEOUS RATE IMMUNOASSAYS

## Field of the Invention

This invention is related to immunoassays and more particularly provides methods for calibrating extended surfaces such as those of microwells used in homogeneous rate immunoassays.

## Background of the Invention

Immunoassays refer generally to a generic class of methods for the detection of ligands or ligand binding partners in fluids such as body fluid samples including urine, blood, cerebrospinal fluid, components thereof and the like. Such techniques rely upon the specificity of reaction between antibodies and antigens or haptens, hereafter ligand binding partners and ligands respectively and interchangably. As is known, the detection of such substances in body fluid samples provides clinical indicia of the state of health of the individual and assists in the diagnosis and prognosis of various disease states.

Accordingly, there is constant technological pressure to develop assays of increasing sensitivity and specificity in order to provide the clinically relevant information as early as possible. Coupled with these pressures are those for reducing the cost such as those incurred in performing repeated assays of the same sample. Repetition of an assay is frequently carried out to guard against one or more components of the assay lacking sufficient quality to produce a trustworthy result.

Many immunoassays rely upon the use of extended solid surfaces having a biologically active molecule, i.e. either the ligand or ligand binding partner or other type of receptor immobilized thereon for capturing from solution onto the solid surface the ligand or ligand binding partner to be detected. Extended surfaces, for purposes of this discussion shall be defined to mean all nonparticulate surfaces, e.g. all surfaces excluding those associated with large numbers of beads such as latex particles and the like. Such extended surfaces would, therefore, include for example, the surfaces of microtiter-type wells, surfaces of paddles, test tubes, microscope slides or other such surfaces regardless of their individual composition or shape.

For example, in a typical sandwich assay for hCG in urine, the surface may have immobilized thereon an antibody, of either polyclonal or monoclonal origin, for specific capturing reaction with a first epitope of the hCG. Thereafter, a second antibody also reactive with the hCG but at a second epitopic site and having a detectable label associated is permitted to react with the captured hCG. Alternatively, the solution phase reactions may be performed prior to or simultaneous with the solid phase or capturing reaction. The label captured on the surface or, removed from the solution phase, may then be detected and quantitatively or qualitatively related to the presence of hCG in the original sample.

A common difficulty associated with such extended surfaces however, results from the technical difficulties related to the immobilization (coating, attachment, or application) of the biologically active molecule on such surfaces. To date, immobilization of such molecules on extended surfaces has produced surface coatings of molecules with inconsistent binding strength and specificity. Further, the surface material often manifests physical inhomogeneities which cause inconsistencies in measuring interactions at the surface or through the surface.

It is another aspect of the present invention to provide methods for substantially obviating the effects of surface to surface variations in the binding activity or specificity of immobilized biologically active molecules.

It is an aspect of the present invention to provide methods for identifying and/or substantially eliminating the effects of physical inhomogeneities which can be associated with the material forming an extended surface used in immunoassays.

It will be recognized that few of the immunoassays employing extended surfaces do so in a homogeneous manner, i.e. one employing no manipulations resulting in separations of the aqueous and solid phases. Those which do employ separation steps, e.g. by dip stick methods which are moved from solution to solution, centrifugation, and the like, are termed heterogeneous. Heterogeneous immunoassays are generally less preferred due to the additional manipulations required by the separation steps since such are time consuming and can potentially introduce errors. Unfortunately, homogeneous assays employing extended surfaces are difficult to perform at the sensitivity levels normally required because of the inherent difficulties in ignoring the labeled materials which have not been captured on the extended surface.

It is desired to employ homogeneous immunoassays so as to reduce the number of manipulative steps required. It is also desirous to implement rate immunoassays so as to achieve an earlier result than could otherwise be effected by waiting for the endpoint to occur. The combination of a homogeneous and rate immunoassays has been commonly achieved by the utilization of solution phase reactions or reactions taking place on the surface of particulate matter dispersed in a solution phase. The combination of a homogeneous and rate immunoassay has not commonly been achieved on extended solid surfaces. One of the predominant reasons for this lack of achievement is due to the difficulty in compensating for surface to surface variations.

It is yet another aspect of the present invention to provide methods for overcoming the effects of surface to surface inhomogeneities associated with extended surfaces whereby homogeneous immunoassays utilizing extended surfaces and performed on a kinetic basis, e.g. rate immunoassays, can be performed with greater accuracy.

It is still yet another aspect to provide methods of standardizing the activity of an extended surface prior to performing an immunoassay on the same surface whereby the immunoassay results may be corrected.

## Summary of the Invention

In accordance with the principles and aspects of the instant invention, there are provided methods for identifying and correcting for inconsistencies in either or both the coating of biologically active materials on surfaces and the effects of physical inhomogeneities associated with the extended surface itself. The methods involve the performance of a calibration assay on the extended surface in question ideally prior to performing the sample immunoassay.

To be most useful, the calibration method of the instant invention advantageously avoids expending or exhausting the activity of the biologically active molecule on the extended surface thereby permitting subsequent performance of the actual assay on the same extended surface. The method advantageously applies to kinetic assay and generally involves the addition of a known amount of calibration reagent ideally prior to the performance of the sample assay. While the calibration reagent and calibration assay could be performed after sample addition, this is less preferred as a high range sample could exhaust the surface prior to the addition of the calibration reagent.

With kinetic or rate immunoassays, the preferred method provides for the addition of a calibration reagent of known activity which should then become detectably captured by the immobilized biologically active molecule on the extended surface. This addition is performed prior to the sample addition whereby the activity and uniformity of the immobilized biologically active molecule on the extended surface may be assessed prior to performance of the sample immunoassay on the same extended surface. The amount of calibration reagent used must be sufficiently low so that the biologically active molecule immobilized on the extended surface is not exhausted by the calibration reaction. The calibration reagent need not use a different label than that employed in the sample immunoassay when the calibration reagent is performed prior to the sample assay or, less preferably, subsequent to the sample assay.

## Detailed Description of the Invention and Best Mode

Methods of the instant invention are advantageously useful with a variety of different immunoassay systems employing extended surfaces for the capture of ligands or ligand binding partners from aqueous samples. As is known, various techniques have been derived for identifying the occurrence of such capture reactions. Generally, such methods rely upon the direct or indirect attachment of a label to the product formed by the capture reaction. The variety of applicable labels has spawned a diverse family of detection techniques that include, for example, the detection of captured fluorescent labels on the extended surfaces, the measurement of altered optical parameters including disruptions in light scatter transmissive qualities, reflectance qualities, and the detection of enzyme labels by the level of their substrate to detectable product conversion capacity (see for example, copending European patent application. No. (claiming priority from USSN 879 236 - ORD 64) filed contemporaneously herewith; Kamentsky U.S. patent NO. 4,487,839; Kronick et al. U.S. patent No. 3,939,350; Elings et al. U.S. patent No. 4,557,861). The general level of knowledge regarding such various techniques is of such an advanced state, that it need not be discussed here in detail. The skilled practitioner will, however, readily perceive the applicability of the instant methods to all such techniques to the extent they rely upon extended surfaces having an active component associated therewith.

The instant invention is most useful for kinetic or rate studies. With such immunoassays, one performs, ideally prior to the assay for the ligand or ligand binding partner, substantially the same immunoassay procedure with a calibration reagent comprising a known level of the ligand or ligand binding partner to be determined which is either labeled directly, or indirectly with additional assay reagents. Again, only small amounts of the calibration materials are ideally used so that the activity of the extended surface is not significantly reduced by such reactions prior to the performance of the sample assay.

Typically, and utilizing an hCG assay as an example, a conventional rate assay would be performed by adding the sample suspected of containing the hCG to be detected along with a labeling reagent to the extended surface, such as a microwell, under conditions suitable for a traditional sandwich assay. After a short waiting period (generally less than about 5 minutes) whereby bubbles and other gross inhomogeneities due to the mixing and addition of the reagents are cleared, one begins measuring the accumulation of label onto the surface either continuously or through a plurality of consecutive measurements. With the progression of time, a series of data points are accumulated allowing the calculation of a curve, such as by standard curve fitting techniques, and the determination of its respective slope. Typically, the slope and/or interpolation of the endpoint of the curve may be utilized separately or in conjunction to make quantitative or qualitative determinations of the hCG in the sample. Such rate immunoassays are especially preferred when performance of conventional endpoint immunoassays would be too time consuming, a situation which occurs with certain ligands or ligand binding partners particularly especially when they are present in very low concentrations.

As may be appreciated, small differences in volumes of sample or reagent addition to the microwell or other extended surface. small differences in activity levels of immobilized active biological molecules on the surface of the microwell, variations induced by unobservable differences in the mixing, incubation, or other mechanical steps involved in the immunoassay, as well as physical inhomogeneities in the microwell material itself can all individually or collectively form sources of error with respect to the sample immunoassay determination.

Accordingly, to overcome such problems and/or at least identify those egregious instances of insurmountable extended surface failures the ideal rate homogeneous immunoassay will now employ the calibration method of the instant invention by first performing a calibration assay utilizing a calibration reagent before performing a sample assay on the same extended surface. Such a calibration reagent, in the circumstances of the hCG example, might include a known amount of labeled hCG or, a known amount of hCG and a known amount of labeling reagent. These calibration materials are added to the extended surface, e.g. a well, under substantially the same conditions as those under which the sample assay itself will be subsequently performed. As earlier indicated, the calibration reagent will be ideally performed prior to the sample assay, however, there are circumstances when the calibration reagent can be performed following the sample assay or, less preferably, simultaneously with the sample assay providing detectably different labels are used so that the calibration and sample assay may be individually monitored. Measurements of captured label are then taken for a period of time from which a rate may then be calculated by conventional curve fitting techniques. Since the amount of calibrator reagent added is known, the calculated slopes or other rate indicia may then be correlated with the known activity thereby standardizing the activity of that particular extended surface.

Thereafter the sample is added to the same extended surface or microwell and the standard label capture rate measurements again made. Final ligand or ligand binding partner determination will then preferably incorporate the correcting factors determined during the calibration assay to derive accurate sample assay results. Under the conditions of not having exhausted the extended surface which may otherwise introduce nonlinearities, well known mathematical methods for applying linear correction factors will be applied to the sample assay based on the results of the calibration assay. For example, should the calibration assay yield a rate that is 10% below that observed from a standard, then the sample assay results from that same surface should be increased by 10%. The calibration, of course, will be specific for each well thereby making all results from all wells comparable despite inter-well specificity, activity, or physical differences.

The amount of calibration reagent added will be optimally adjusted to a known amount or activity level taking into consideration the maximum activity level generally expected from the extended surface, the range of levels of the ligand or ligand binding partner to be expected in the sample, and the amount of labeled calibration reagent which must be immobilized on the surface to be facilely detected. Naturally, one would preferably wish to diminish if not avoid the effects of any competition between the calibration reagent and the sample ligand or ligand binding partner for immobilized binding sites thereby guiding the adjustment of calibration activity accordingly, particularly if the sample and calibration assays are performed simultaneously. Alternatively one may, based on suitably constructed standards incorporate the effect of such competition in the correction of the sample immunoassay results.

The method will advantageously allow the identification of those wells having egregious physical, specificity or activity inhomogeneities and elimination thereof in those cases when the error, introduced by any such inhomogeneities, exceeds a permissible level during the calibration assay. Suitable alarms may be incorporated to direct the avoidance of that particular well by the technician. if manual, or by the instrument, if automated.

The following synopsizes the preferred procedure for performing a rate homogeneous immunoassay employing an extended surface and utilizing the calibration methods of the instant invention. An extended surface having an immobilized biologically active molecule is provided. The biologically active molecule is expected to be specifically reactive with its binding partner, a ligand or ligand binding partner present in the sample and for such material present within known amounts in the calibration reagent. Calibration reagent comprising a known amount of ligand or ligand binding partner or their respective simulacra is contacted with the extended surface under conditions suitably conducive for an immunoassay. The calibration reagent will further comprise a label directly associated with said ligand or ligand binding partner, or if indirectly associated such as occurs when labeling is by way of attachment through additional ligand or ligand binding partner reactions, then the addition of such additional labeled calibration reagent. A waiting period of approximately 5 minutes is allowed to elapse to permit bubbles and other mixing inhomogeneities to disappear. It will be recognized that the waiting period may be adjusted when it can be determined that the signal associated with the detection of captured label can be distinguished from the noise background. Such signal sampling and signal conditioning aspects to improve signal-to-noise ratios are also matters understood by those skilled in the art. Following the waiting period, the signal is monitored continuously, or at a sampling rate sufficient to permit interpolation therebetween so that conventional curve fitting or similar procedures may be instituted and a slope or rate determined therefrom. That rate will be dependent upon the level of ligand or ligand binding partner in the calibration reagent and also will reflect the activity, specificity, and physical perfection of the extended surface under test. Sample and labeling reagent is contacted with the extended surface under substantially the same immunoassay conditions. (Alternately, the labeling reagent may be added to the surface before the sample, or previously mixed with the sample and the mixture added to the surface.) After another waiting period, captured label is detected, again on a continuous or intermittent basis for a time period sufficient to permit the application of curve fitting techniques and the determination of the sample rate. By a variety of mathematical manipulations available, the calibration rate associated with the known level of ligand may be used to correct the rate determined in the sample assay in order to derive an accurate assay of the ligand or ligand binding partner in the sample.

While the methods of the instant invention have been described in terms of an hCG example, one will readily understand that the methods are by no means so limited. They may be used with virtually any homogeneous rate assay for the detection of any ligand or ligand binding partner utilizing an extended surface and for which a simulacrum molecule, e.g. a molecule of substantially similar binding characteristics, may be obtained and labeled, either directly or indirectly, for use as a calibration reagent. Similarly, while particular labels have been mentioned, the invention may be readily extended to employment with all types of labels, the only limitation being that they be detectable. These and other substitutions, including the choice of order of performance of the calibration assay with relation to the sample immunoassay, the reagents to be employed comprising the calibration assay and the actual mathematical correction manipulations utilized, will now be apparent to those readily skilled in the art. All such modifications shall be deemed equivalents and within the scope of the principles and claims of the instant invention.

**Claims**

1. A method for use in an immunoassay utilizing a labeled component and employing an extended surface having an immobilized biologically active molecule disposed thereon for the detection of a ligand or a ligand binding partner specifically reactive therewith, which ligand or ligand binding partner is previously or subsequently tagged with a detectable label, said method comprising the step of performing a calibration assay on the same extended surface on which said immunoassay shall be conducted, said calibration assay employing a known activity level of calibration reagent whereby the activity level and/or distribution thereof on the extended surface may be assessed.

2. The method as provided in Claim 1 wherein the activity level of the extended surface is employed to correct the results of the immunoassay.

3. The method as provided in Claim 1 or Claim 2 wherein the immunoassay is a rate immunoassay and the calibration assay is performed prior to said rate immunoassay.

4. A method for qualifying an extended surface having an immobilized biologically active molecule in a sample immunoassay comprising the step of performing a calibration assay prior to said sample immunoassay on said extended surface wherein said calibration assay includes a calibration reagent having a known level of activity and capable of binding to said immobilized biologically active molecule if active while still permitting a sample immunoassay to be performed using the same extended surface.

5. The method of Claim 4 wherein said calibration assay and said sample assay are rate determination assays.

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| X | EP-A-0 112 721  (COMTECH RESEARCH UNIT LTD.) * page 5, lines 3-8; claims 1-3, 33-38 * | 1,2,4 | G 01 N   33/557 G 01 N   33/543 |
| A | | 3,5 | |
| A | DE-A-3 122 305  (V.F. NICULESCU) * claim 1 * | 1,2,4 | |
| A | US-A-4 558 013  (V.A. MARINKOVICH) * abstract; claim 1 * | 1,2,4 | |
| A | WO-A-8 400 779  (EASTMAN KODAK CO.) * page 14, line 15 - page 15, line 18; claims * | 3 | |
| A | EP-A-0 177 352  (THE STATE OF VICTORIA) * abstract; claims 1-12 * | 1,2,4 | TECHNICAL FIELDS SEARCHED (Int. Cl 4)  G 01 N   33/00 |
| A | EP-A-0 093 613  (SYVA CO.) * pages 2, 3; claims 1, 12, 17, 18 * | 1,2,4 | |

The present search report has been drawn up for all claims

| Place of search | Date of completion of the search | Examiner |
|---|---|---|
| BERLIN | 14-08-1987 | DE KOK A.J. |